# EUROPEAN PATENT APPLICATION

(11) **EP 4 276 173 A1**
(43) Date of publication of application: **15.11.2023**
(21) Application number: 22188710.2
(22) Date of filing: 04.08.2022
(51) Int. Cl.: C12N 5/078, C12N 5/00

(54) **STABLE HUMAN PLATELET LYSATE COMPOSITION, METHODS AND USES THEREOF**

(30) Priority: 10.05.2022 PT 2022117969
(71) Applicant: Stemmatters, Biotecnologia e Medicina Regenerativa, S.A., 4805-017 Barco GMR (PT); PL BioScience GmbH, 52068 Aachen (DE)
(72) Inventor: MOUTINHO DA COSTA, PEDRO MIGUEL, 4805-017 BARCO (PT); ANDRÉ DE SOUSA MOREIRA, ELSA FÁTIMA, 4805-017 BARCO (PT); ROMERO AMANDI DE SOUSA, RUI PEDRO, 4805-017 BARCO (PT); HEMEDA, HATIM, 52068 Aachen (DE)
(74) Representative: Patentree

(57) **Abstract**

The present disclosure relates to a stable human platelet lysate composition; preferably a stable heparin-free human platelet lysate composition; methods for obtaining said composition and uses thereof.

## Description

### TECHNICAL FIELD

The present disclosure relates to stable human platelet lysate composition; methods for obtaining said composition and uses thereof.

### BACKGROUND

The demand for standardised and safe culture systems, including cell culture supplements, is rising rapidly and globally. In this regard, human platelet lysate (hPL) has attracted significant attention as raw/ancilliary material for biotechnology applications (at both research and clinical levels). Prepared from human plasma or platelet concentrates, hPL contains high concentration of growth factors that enhance cell proliferation, with superior performance compared to fetal bovine serum (FBS), the gold standard for cell culture supplementation.

While the requirements for basic research are low, clinical cell manufacturing has high normative and compliance requirements. Increasing progress and clinical translation in cell therapy demands xeno-free cell culture media produced according to GMP standards. Although hPL is a human-derived product, its wider use is limited by high fibrinogen content which, in combination with calcium present in the cell culture media, induces formation of fibrin clots, thus requiring addition of heparin (an anticoagulant) to cell culture media supplemented with hPL (to prevent coagulation). The use of porcine-derived heparin renders a non-xeno-free cell culture process. In this case, heparin supplementation can negatively impact the quality of the product, as (i) heparin might directly influence cell growth due to interaction with several growth factors, and (ii) recombinant heparin (i.e., an alternative to the porcine reagent) is not as efficient in preventing the formation of clots (namely small clots that form upon thawing of hPL), and (iii) the addition of heparin constitutes an extra step in the preparation of the cell culture media [1]. Therefore, to provide a more user-friendly and improved quality cell culture supplement, fibrinogen should be removed from the final formulation.

The most efficient mechanism to remove fibrinogen from hPL is the addition of Ca²⁺ (in the form of CaCl₂, at a final concentration of 20 mM) during the manufacturing process, to induce fibrin formation and consequent coagulation [2]. This method ensures that the majority of the fibrinogen, as well as other coagulation factors, are removed (which is confirmed by the absence of coagulation when used as supplement of cell culture media), while having a reduced impact on the profile and content of growth factors of the resulting hPL product.

Ideally, the method for removal of the fibrinogen content in hPL should be carried out below maximum phisiological temperatures to preserve the content and quality of growth factors, minimize or even avoid the use of reagents of animal or recombinant origin, render a product free of visible particles and low subvisble particles, as well as capable of meeting industry and normative standards, including those of good manufacturing practices.

Document EP2723354 [3] describes a method for preparing an hPL composition, comprising the steps of (a) lysing platelets providing a lysate; (b) removing cell debris; and (c) depleting fibrinogen by forming a removable mass by adding a metal salt such as calcium chloride and a composition comprising heparin-containing platelet lysates depleted of fibrinogen.

Document EP3638263 [4] relates to a process for obtaining a pooled human platelet lysate, the pooled human platelet lysate itself and its use for treating neurological disorders such as neurodegenerative, neuroinflammatory, neurodevelopmental and/or neurovascular disorders. EP3638263 relates to a process for preparing a heat-treated pooled human platelet lysate, said process comprising the steps of: a) providing a pooled human platelet lysate (pHPL), b) heat-treating the pooled human platelet lysate at a temperature of 50°C to 70°C during 20 to 40 minutes, and c) purifying the heat-treated pooled human platelet lysate of step b) using glass beads and CaCl₂.

Document DOI: 10.1039/C9TB01764J [5] describes a process for obtaining pooled platelet lysate, being the pooled human platelet lysate itself used in media for human mesenchymal stem cell expansion/culture. DOI: 10.1039/C9TB01764J [5] describes a process comprising the steps: a) freezing and thawing cycles (-80 °C/37 °C) and centrifugation (3000 x g for 30 minutes at 20°C, b) platelet activation with calcium chloride (23 mM) in the presence of glass beads at 24-26 °C and removal of fibrin, and c) centrifugation of the suspension and heat treatment of the supernatant at 56 °C for 30 minutes followed by centrifugation to isolate insoluble proteins.

However, document EP3638263 [4] adopts heparin, while EP3638263 [4] and DOI: 10.1039/C9TB01764J [5] adopt heat treatment of hPL to reduce fibrinogen content. EP3638263 [4] and DOI: 10.1039/C9TB01764J [5] do not report critical quality attributes of hPL, including particulate matter and growth factors' composition.

These facts are disclosed to illustrate the technical problem addressed by the present disclosure.

### GENERAL DESCRIPTION

The present disclosure relates to a method for production of a xeno-free human platelet lysate (further referred as XF-hPL). Using the method described in the present disclosure, it is possible to produce an hPL product with a low fibrinogen content, high growth factor content and quality, capable of meeting performance and regulatory requirements either as a raw material for cell expansion or advanced therapy medicinal products' manufacturing, or for manufacturing of biological medicinal product.

A stable human platelet lysate composition comprising less than 5 µg/ml of fibrinogen and a mass ratio of PDGF-BB growth factor/fibrinogen content not less than 6 ng/µg. Surprisingly the composition of the present disclosure avoids the need of heparin addition because does not coagulate and/or does not induce clot formation. The composition of the present disclosure is suitable to be used as an improved cell culture medium, as a heparin-free composition.

In an embodiment, the composition of the present disclosure is a heparin-free composition.

In an embodiment, the composition of the present disclosure is a xeno-free composition.

In an embodiment, the composition of the present disclosure may comprise less than 4 µg/ml of fibrinogen; preferably less than 3 µg/ml of fibrinogen.

In an embodiment, the mass ratio of PDGF-BB growth factor/fibrinogen content is not less than 8 ng/µg; preferably not less than 10 ng/µg.

In an embodiment, the growth factor may be selected from a list consisting of: BDNF, EGF, FGF-2, GM-CSF, HGF, IL-1β, IL-8, PDGF-AB, PDGF-BB, VEGF and combinations thereof.

In an embodiment, the mass ratio of growth factor (nanograms) per microgram of fibrinogen; wherein the growth factor is selected from BDNF, EGF, VEGF-A, or combinations thereof; ranges from 0.5-2.0 ng_{growth factor}/µg_{fibrinogen}, preferably 1.0-1.5 nggrowth _{factor}/µg_{fibrinogen}.

In an embodiment, the mass ratio of growth factor (nanograms) per microgram of fibrinogen; wherein the growth factor is selected from FGF-2, GM-CSF, HGF, IL-1β, or combinations thereof; ranges from 0.05-0.5 ng_{growth factor}/µg_{fibrinogen}, preferably 0.1-0.4 nggrowth _{factor}/µg_{fibrinogen}.

In an embodiment, the mass ratio of growth factor (nanograms) per microgram of fibrinogen; wherein the growth factor is selected from PDGF-AB, PDGF-BB, or combinations thereof; ranges from 4-12 ng_{growth factor}/µg_{fibrinogen}, preferably 6-8 ng_{growth factor}/µg_{fibrinogen}.

In an embodiment, the composition of the present disclosure may comprise a ratio of growth factor (nanograms) per microgram of fibrinogen ranging between 0.5-2.0 ng_{growth factor}/µg_{fibrinogen}, (wherein the growth factor is BDNF, EGF, VEGF-A), 0.05-0.5 ng_{growth factor}/µg_{fibrinogen}, (wherein the growth factor is FGF-2, GM-CSF, HGF, IL-1β), and 4-12 ng_{growth factor}/µg_{fibrinogen}, (wherein the growth factor is PDGF-AB, PDGF-BB), preferably from 1.0-1.5 ng_{growth factor}/µg_{fibrinogen}, ( wherein the growth factor is BDNF, EGF, VEGF-A), 0.1-0.4 ng_{growth factor}/µg_{fibrinogen}, (wherein the growth factor is FGF-2, GM-CSF, HGF, IL-1β), and 6.0-8.0 ng_{growth factor}/µg_{fibrinogen}, (wherein the growth factor is PDGF-AB, PDGF-BB).

In an embodiment, the composition of the present disclosure has a clear yellow colour, without visible particles detected by visual observation, more preferably without insoluble visible particles.

In an embodiment, the composition of the present disclosure may comprise a clarity and opalescence degree, measured by nephelometry, less than 400 NTU; preferably less than 350 NTU; more preferably less than 300 NTU.

In an embodiment, the composition of the present disclosure may comprise a clarity and opalescence degree measured by nephelometry ranges from 100-350 NTU; more preferably 150 - 200 NTU.

In an embodiment, the composition of the present disclosure may comprise sub-visible particles, measured by Flow Imaging Microscopy (FIM), the number of sub-visible particles up to 25 µm in size in the composition of the present disclosure being inferior to 2.0 × 10⁷ particles/mL, preferably inferior to 1.0 × 10⁷ particles/mL, more preferably inferior to 5.0 × 10⁶ particles/mL.

In an embodiment, the composition of the present disclosure may comprise sub-visible particles, measured by Flow Imaging Microscopy (FIM), the number of particles with a size below 25 µm is 5.0 × 10⁶ particles/mL. In another embodiment, the number of particles with a size below 5 µm in the composition of the present disclosure is 3.9×10⁶ particles/mL. In another embodiment, the composition of the present disclosure comprises an amount of sub-visible particles with size from 5 - 10 µm below 1.3 × 10⁵ of particles/mL.

In an embodiment, the composition of the present disclosure may comprise undetectable amounts of fibrinogen beta chains, as measured by proteomics analysis using LC-MS methodology.

In an embodiment, the composition of the present disclosure may comprise stable storage up to 6 months in refrigerated conditions at -80°C to 5°C, preferably - 20°C to 4°C.

Another aspect of the present disclosure relates to a cell culture medium comprising the stable human platelet lysate composition of the present disclosure.

Another aspect of the present disclosure relates to a medicinal product incorporating the stable human platelet lysate composition of the present disclosure.

Another aspect of the present disclosure relates to a method for obtaining a stable human platelet lysate comprising the following steps:
- obtaining frozen human platelet units;
- submitting said human platelet units to thawing / freezing cycles to disrupt the platelets' membrane;
- adding silica and calcium chloride to the mixture obtained in the previous step to induce clot formation;
- incubating the sample at a temperature ranging from 35 to 39°C, for up to 1 to 4 hours;
- manual disruption of the clot;
- centrifugation of the samples and collection of the obtained supernatant;
- filtering the supernatant to obtain a stable human platelet lysate; preferably a stable heparin-free human platelet lysate.

In an embodiment, adding 0.005 - 0.04 % (m/v) of silica combined with 0.5 - 1.0 % of calcium chloride.

Another aspect of the present disclosure relates to heparin-free human platelet lysate obtainable by the method of the present disclosure.

In an embodiment, the method disclosed in the present disclosure results in an improved XF-hPL as compared to the state of the art. Namely, the product is clear and free of visible particulate matter, the fibrinogen content is consistently below 5 µg/mL, with fibrinogen beta chains undetectable by proteomic analysis; the final product does not coagulate once reconstituted, i.e., it is easier to manipulate; it is devoid of heparin during manufacturing or reconstitution; and it is devoid of heparin replacements or any additional animal derived component.

In an embodiment, the starting material for XF-hPL preparation comprises human platelet units (hPUs), including hPUs which no longer can be used for transfusion purposes, stored at - 80°C to - 20°C, and the following reagents are added to the pooled starting material during the manufacturing process: calcium chloride (aqueous solution, 1 M concentration); and silica (high-purity grade, containing 3-7% (m/m) of water).

In an embodiment, the stable human platelet lysate composition of the present disclosure, may be obtained by the methods disclosed, and is:
- a xeno-free product, devoided of heparin or any other animal-derived substitutes;
- with a very low fibrinogen content, as result of a fibrinogen removal process;
- with a high content of growth factors, at similar or superior levels compared to a non-xeno-free product or other commercially-available xeno-free products;
- with undetectable visible particles and reduced levels sub-visible particles, lower than those of other commercially-available xeno-free products.

### BRIEF DESCRIPTION OF THE DRAWINGS

The following figures provide preferred embodiments for illustrating the disclosure and should not be seen as limiting the scope of invention.
**Figure 1****:** Representative image of primary packaging used for filling of XF-hPL.
**Figure 2****:** Illustration of an embodiment of results of quality control of the XF-hPL final product - analytical parameters. Characterisation included total protein content, osmolality, pH, fibrinogen content, and concentration of growth factors EGF (Epidermal growth factor) and PDGF-AB (Platelet-Derived Growth Factor-AB). Values represent the mean ± standard deviation of at least three different lots. Dashed lines represent the range defined in the QC panel.
**Figure 3****:** Illustration of an embodiment of results of quality control of the XF-hPL final product - growth factor content via bead-based multiplex assay.
**Figure 4****:** Illustration of an embodiment of results of quality control of the XF-hPL final product - growth factor content normalised for fibrinogen content in the sample.
**Figure 5****:** Illustration of an embodiment of results of quality control of the XF-hPL final product - product performance. Cell proliferation, measured by DNA quantification via PicoGreen assay, for hASCs and HFF-1 cells cultured for 7 days in media supplemented with XF-hPL (5 % v/v; representative lots) or FBS (10 % v/v). Values represent the mean ± standard deviation of at least three (3) different lots. Statistical analysis using Kruskal-Wallis test followed by Dunn's multiple comparisons test. ^{∗} p < 0.05, compared to hASCs cultured in media supplemented with 10 % FBS.
**Figure 6****:** Illustration of an embodiment of results of product stability under accelerated conditions (4°C), long-term storage conditions (-20°C, -80°C) and for different periods of time (1, 3 and 6 months). Dashed lines represent the range defined in the QC panel.

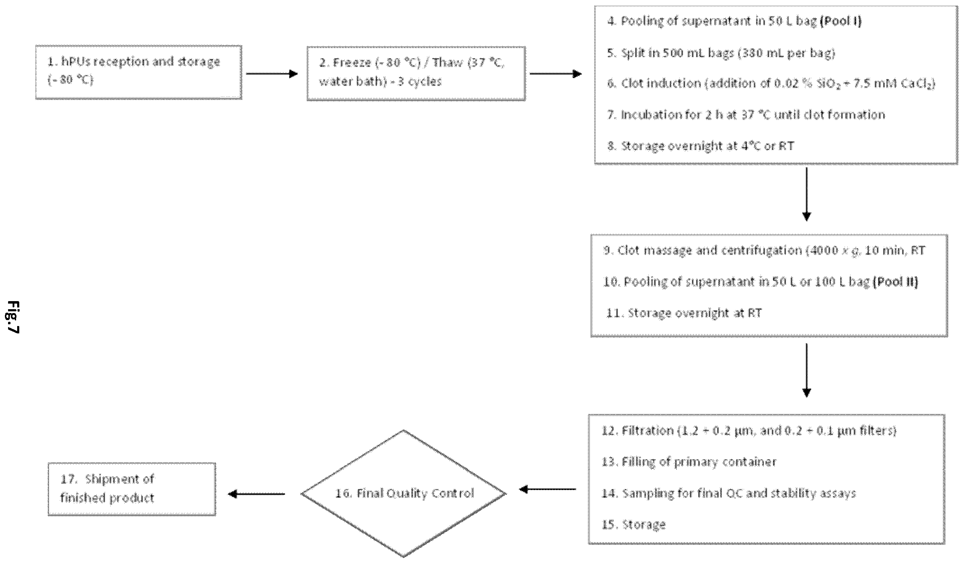

**Figure 7****:** Illustration of the process for manufacturing of XF-hPL.

### DETAILED DESCRIPTION

The present disclosure relates to a stable human platelet lysate composition; preferably a stable heparin-free human platelet lysate composition; methods for obtaining said composition and uses thereof.

In an embodiment, the hPUs undergo up to three freeze-thaw cycles (at -80°C and 37°C, respectively) to disrupt the platelets' membranes and release the cytoplasmatic and granules content. The hPUs are then transferred and pooled to a single-use sterile container (e.g., high volume sterile bag), using a plasma manual processor, and samples are collected for in-process control, including quantification of fibrinogen (competitive ELISA assay) and total protein content (BCA assay).

In an embodiment. after the control analysis, the pooled hPL is split into smaller individual single-use sterile bags.

In an embodiment, calcium chloride and silica are added to the pooled hPL to induce clot formation. The formation of a uniform clot is visually confirmed. In a further embodiment, the final concentration of calcium chloride in the hPL solution ranges from 5.0 to 10 mM, preferably from 6.0 to 8.0 mM, more preferably is 7.5 mM; and the final concentration of silica in the hPL solution ranges from 0.005 to 0.04 % (w/v), preferably 0.01 to 0.03 % (w/v), more preferably is 0.02 % (w/v).

In an embodiment, the addition of calcium chloride and silica is followed by incubation at a temperature ranging from 35 to 39°C, preferably 37°C, for up to 1 to 4 hours, preferably 2 hours, more preferably 1.5 hours.

In an embodiment, the clotted material is stored at a temperature ranging between 2 and 25°C, preferably 15-23°C, for a period of up to 16 to 20 hours.

In an embodiment, the clotted material is subjected to manual disruption and centrifugation at 3500 to 5000 x *g,* preferably 4000 x *g,* for 10-20 minutes, preferably 15 minutes, at 15-25°C. In a further embodiment, the resulting supernatant is collected and pooled onto a single-use sterile bag; samples are collected for in-process control analysis, such as quantification of fibrinogen (competitive ELISA assay), total protein content (BCA assay), and assessment of sterility (BacT/Alert microbial detection system).

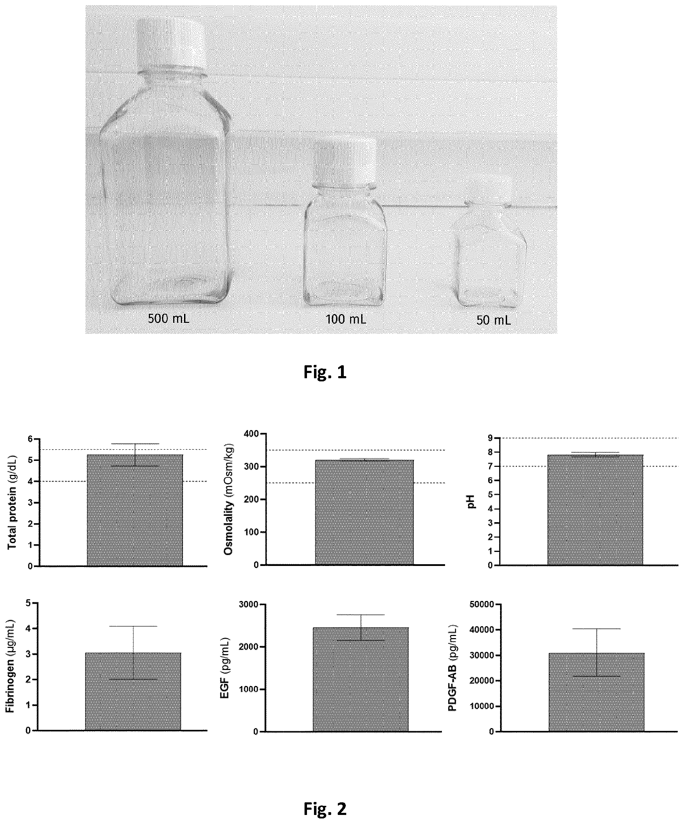

In an embodiment, the pooled supernatant is subjected to terminal sterile filtration, preferably by filtration, more preferably by using a 0.22 µm filter (removal of bacteria with cell wall) and a 0.1 µm filter (removal of mycoplasma, i.e., bacteria without cell wall). The sterile material is then stored in a primary packaging (Fig. 1) at - 80°C to -20°C, preferably -72 to -78°C, until further use.

In an embodiment, the appearance of the final product was visually assessed (i) after filling in the primary packaging and (ii) after storage at -80°C and thawing at room temperature, in accordance with the European Pharmacopeia (Eur. Ph.) 2.2.1, Clarity and degree of opalescence of liquids (Visual method), and 2.9.20, Particle contamination: Visible particles.

In an embodiment, the clarity and degree of opalescence of the final product was assessed *via* measurement of nephelometry, in accordance with Eur. Ph. 2.2.1, Clarity and degree of opalescence of liquids (Instrument method). In an embodiment, the presence of sub-visible particles in the final product was assessed by Imaging Flow Microscopy (FlowCam). By providing high-resolution images in real-time along with the particle size, count, shape and other parameters, this method allows for detection and characterisation of each individual particle detected in a sample. The amount of sub-visible particles was quantified for a representative batch of XF-hPL and a commercially-available fibrinogen-depleted xeno-free hPL reference sample (further referred as sample A). The table below shows that XF-hPL presents significantly lower number of subvisible particles as compared to sample A for all particle size ranges, with emphasis to subvisible particle sizes up to 25 µm of size.

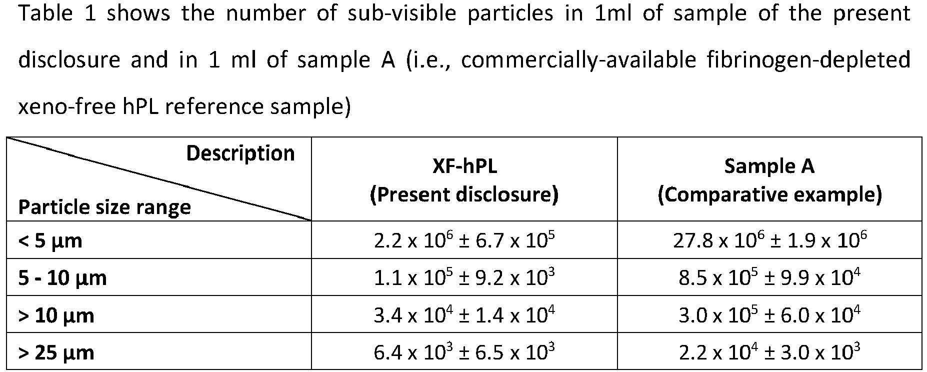

In an embodiment, sterility was assessed by the detection of aerobic/anaerobic organisms in samples of the final product, via sample inoculation in BacT/Alert culture bottles and incubation for 14 days in the BacT/Alert microbial detection system, in accordance with the European Pharmacopeia (Eur. Ph. 5.1.6).

In an embodiment, detection of mycoplasm contamination in samples of the final product was performed by quantitative real-time PCR (qRT-PCR), using the Venor^{®}GeM qEP kit, in accordance with Eur. Ph. 2.6.7.

In an embodiment, detection of endotoxin contamination in samples of the final product was performed via gel-clot assay, in accordance with Eur. Ph. 2.6.14.

In an embodiment, the pH of samples of the final product was measured using a pH meter, in accordance with Eur. Ph. 2.2.3.

In an embodiment, the osmolality levels in samples of the final product were measured using an osmometer, in accordance with Eur. Ph. 2.2.35.

In an embodiment, the total protein content in samples of the final product was measured using the BCA assay (ThermoFisher), in accordance with Eur. Ph. 2.5.33., and USP <1057> Protein Determination.

In an embodiment, the quantification of human growth factors epidermal growth factor (EGF) and platelet-derived growth factor AB (PDGF-AB) in samples of the final product was performed via ELISA assay, in accordance with the manufacturer's instructions.

In an embodiment, the performance of samples of the final product as cell culture media supplement, compared to non-xeno-free hPL and high-grade FBS, was assessed using human adipose-derived stromal/stem-like cells (hASCs) and a human dermal fibroblast cell line (HFF-1). Cell proliferation was measured after expansion for 7 days via quantification of DNA, using the PicoGreen kit.

In an embodiment, fibrinogen quantification was performed via competitive ELISA assay, using a commercially-available kit (Abnova, reference KA0475) allowing detection of fibrinogen in human plasma samples, in accordance with the manufacturer's instructions. Briefly, the kit uses a 96-well microplate (with removable strips), pre-coated with a murine antibody specific for this protein. Fibrinogen in standards and samples competes with a biotinylated human fibrinogen protein sandwiched by the immobilized antibody and streptavidin-peroxidase (SP) conjugate. All unbound material is washed away, and a peroxidase enzyme substrate is added. The colour development is stopped, and the intensity of the colour (proportional to the concentration of fibrinogen) is measured.

In an embodiment, the quantification of human brain-derived neurotrophic factor (BDNF), epidermal growth factor (EGF), basic fibroblast growth factor (FGF-2), granulocyte-macrophage colony-stimulating factor (GM-CSF), hepatocyte growth factor (HGF), interleukin 1β (IL-1β), interleukin 8 (IL-8), platelet-derived growth factor BB (PDGF-BB) and vascular endothelial growth factor A (VEGF-A), in samples of the final product, was performed via bead-based immunoassays, using a 9-Plex human ProcartaPlex multiplex plate (ThermoFisher Scientific), compatible with the MAGPIX Luminex equipment, in accordance with the manufacturer's instructions. ProcartaPlex immunoassays are based on the principles of a sandwich ELISA, using two highly specific antibodies binding to different epitopes of one protein to quantitate all protein targets simultaneously. As opposed to ELISA assays, the capture antibody (specific for a single target protein) is conjugated to a spectrally unique bead (and not adsorbed to the microplate well), and bound proteins are identified with biotinylated antibodies and streptavidin-R-phycoerythrin (RPE). The conjugation of protein-specific antibodies to a distinct bead allows for analysis of multiple targets in a single well.

In an embodiment, the analysis of protein content in samples of the final product was performed by liquid chromatography-mass spectroscopy (LC-MS) [6]. Briefly, samples were diluted in ultra-pure water to a final concentration of 10 µg/mL, mixed with 4x Laemmli sample buffer (Bio-Rad) to a final concentration of 1×, incubated at 95°C for 5 minutes, cooled to room temperature and stored at -20°C. Samples were analyzed on a NanoLC^{™} 425 System (Eksigent) coupled to a Triple TOF^{™} 6600 mass spectrometer (Sciex) and the ionization source (ESI DuoSpray^{™} Source).

In an embodiment, the quantification of silica and calcium in samples of the final product was performed via inductively coupled plasma optical emission spectrometry (ICP-OES). Briefly, samples were digested using sulfuric acid (H₂SO₄) and perchloric acid (HClO₄), followed by hydrofluoric acid (HF). Quantification the digested solution was performed against standard curves of silica and calcium.

In an embodiment, scalability was assessed from small laboratorial scale (50 - 500 mL) up to industrial scale (50 L), with quality control (QC) testing revealing a final product with clear, yellow appearance (without any visible particles or clots), and very consistent analytical parameters between batches (Fig. 2), namely:
- Total protein content: 5.26 ± 0.52 g/dL;
- Osmolality: 320.5 ± 3.6 mOsm/kg;
- pH: 7.83 ± 0.15;
- Epidermal growth factor (EGF) content: 2455 ± 302 pg/mL (ELISA-based assay);
- Platelet-derived growth factor AB (PDGF-AB) content: 31027 ± 9354 pg/mL (ELISA-based assay).

Fibrinogen levels below 5 µg/mL (3.05 ± 1.04 µg/mL) were measured for all batches produced by the described method (Fig. 2), thus confirming that the silica-based method consistently achieves removal of fibrinogen in hPL independently of the production scale. Moreover, residual levels of silica in the final product (~ 3 ppm) were similar to basal plasma levels, thus indicating that clotting and the subsequent processing (including filtration) removes all the silica added during the manufacturing process.

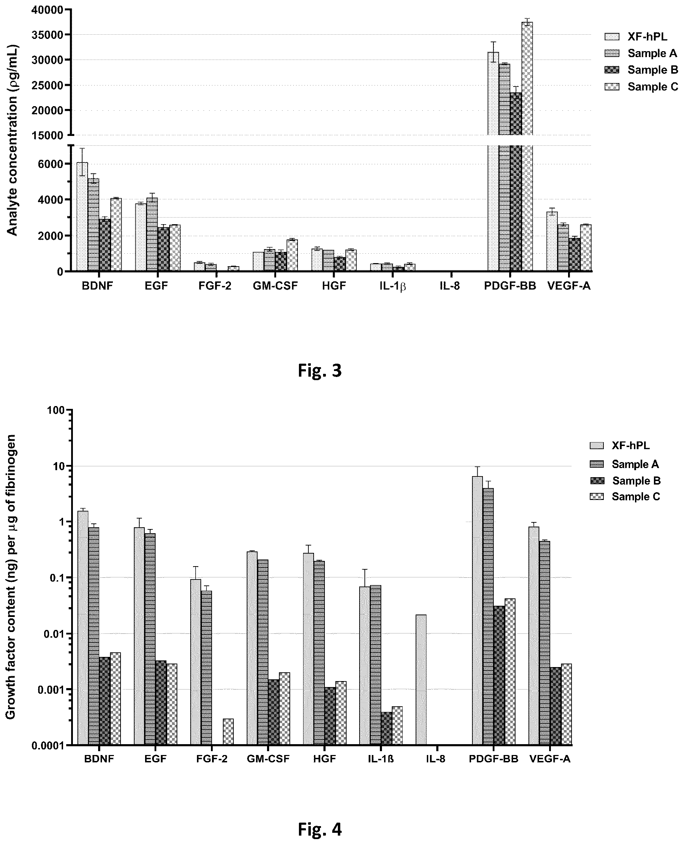

In an embodiment, a total of nine (9) growth factors and cytokines (BDNF, EGF, FGF-2, GM-CSF, HGF, IL-1β, IL-8, PDGF-BB and VEGF-A) were quantified in samples of the final product via bead-based multiplex assays, using a 9-Plex multiplex plate compatible with the MAGPIX Luminex equipment. Different samples were used to compare, namely a commercially-available fibrinogen-depleted xeno-free hPL (sample A), and commercially-available fibrinogen-containing xeno-free hPL (further referred as sample B and sample C). In this regard, the levels of growth factors (Fig. 3), as well as the growth factor content (nanograms) per microgram of fibrinogen (in each sample) (Fig. 4) were found to be similar or superior to those of the reference samples included in the assay. Importantly, when considering the relative fibrinogen level, the proposed method yields a superior concentration of growth factors (Fig. 4).

In an embodiment, absorbance at 410 nm wavelength was determined for 200 µl volume samples of hPL samples using the equipment Synerg HTX and the software GEN5. Optical density was calculated for each sample.

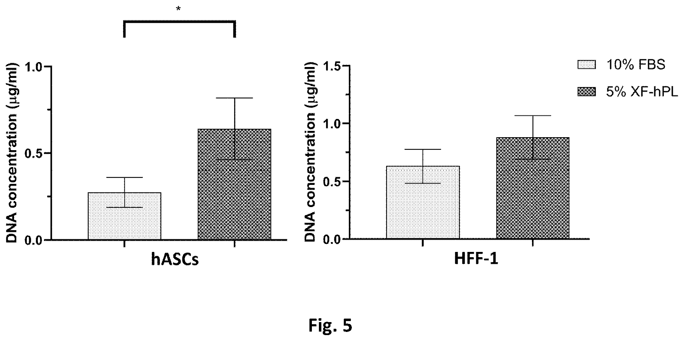

In an embodiment, product characterisation included performance assays with two different cell types - human adipose-derived stem/stromal-like cells (hASCs) and human dermal fibroblasts (HFF-1). Product performance was assessed by measuring the proliferation of hASCs or HFF-1 cells expanded in culture medium supplemented with the XF-hPL (at 5 % v/v), which was compared to that of cells cultured in medium supplemented with 10 % FBS (i.e., the standard supplementation for these cells). Cell proliferation was measured via quantification of DNA extracted from cells in a culture dish (using the Quant-iT^{™} PicoGreen^{™} double-stranded DNA (dsDNA) assay), with the amount of DNA extracted proportional to the total number of cells in the culture surface. As shown in Fig. 5, enhanced proliferation was detected for hASCs and HFF-1 cells cultured for 7 days in media supplemented with 5 % XF-hPL, compared to that detected for cells expanded in media supplemented with 10 % FBS.

In another embodiment, detailed characterisation of protein content provides further analytical data to substantiate the product's biochemical profile. In contrast to chemically-defined media, composition of hPL products is not defined. As manufacturers of cellular therapies are moving towards better defined cell therapy products, a broader and more detailed characterisation of culture media supplements is highly desirable. Therefore, proteomic analysis of XF-hPL samples was carried out by Liquid Chromatography Mass Spectroscopy (LC-MS) [6]; a commercially-available XF-hPL (Sample A) was used as reference for comparison. Immunoglobulin were the most highly represented family of proteins detected (N = 57, including immunoglobulin chains) followed by apolipoproteins and members of the complement cascade (N = 12). Cytoskeletal and cytoskeletal associated proteins (e.g., actin, tubulin, filamin, talin, myosin, vinculin), oxygen and iron/copper transporters (hemoglobin, serotransferin, ceruloplasmin) and albumin were also identified in all samples of final product. Interestingly, while both fibrinogen alpha and beta chains were detected in the reference sample, only the alpha chain was detected in the XF-hPL, suggesting that the residual levels of fibrinogen in the final product (quantified by ELISA assay) are from its alpha chain. The low fibrinogen content, as well as the absence of fibrin in the final product, are expected to enhance the safety profile of XF-hPL, as fibrinogen and fibrin have been linked with inflammatory modulation [7].

Moreover, data analysis allowed identification of proteins present in the XF-hPL at considerably higher levels compared to the reference sample A (ratio > 2), including (i) chaperone proteins (endoplasmic reticulum chaperone BiP, Heat-shock protein HSP90-alpha), (ii) regulators of actin function (coronin-1A), (iii) proteinase inhibitors (kallistatin), (iv) immunoglobulins (immunoglobulin heavy variable 3-43) and (v) integrin-associated proteins (integrin-linked protein kinase).

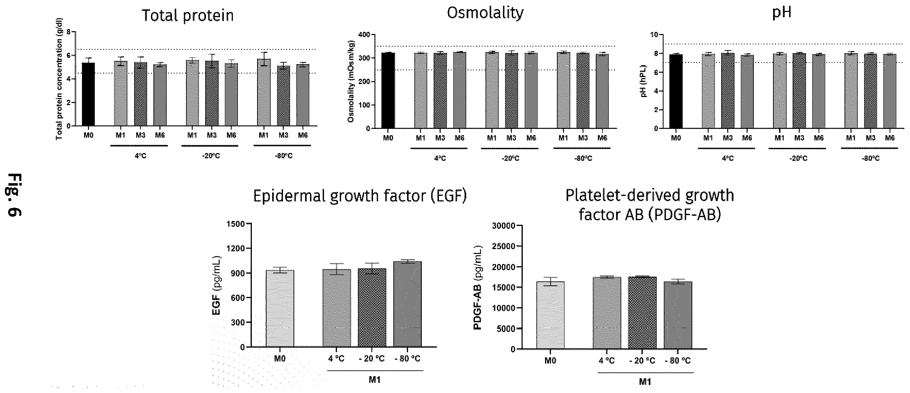

In an embodiment, stability studies have shown that the final XF-hPL product retains its characteristics upon storage for up to 6 months in refrigerated conditions (4°C, -20°C, -80°C), as depicted in Fig. 6.

The results herein presented substantiate a novel method for production of ready-to-use XF-hPL, without use of heparin or any animal-derived substitutes, which yields very low fibrinogen content, high growth factor/fibrinogen content ratio and superior performance as a cell culture supplement.

The term "comprising" whenever used in this document is intended to indicate the presence of stated features, integers, steps, components, but not to preclude the presence or addition of one or more other features, integers, steps, components or groups thereof.

The disclosure should not be seen in any way restricted to the embodiments described and a person with ordinary skill in the art will foresee many possibilities to modifications thereof. The above-described embodiments are combinable.

The following claims further set out particular embodiments of the disclosure.

### References

[1] Khorana AA, Sahni A, Altland OD, Francis CW. Heparin Inhibition of Endothelial Cell Proliferation and Organization Is Dependent on Molecular Weight. Arterioscler Thromb Vasc Biol. 2003 Nov 1; 23(11):2110-5; doi: 10.1161/01.ATV.0000090671.56682.D7.
[2] US 9688959B2, Compositions, uses, and preparation of platelet lysates.
[3] EP2723354, Compositions, uses, and preparation of platelet lysates.
[4] EP3638263, Process for preparing a pooled human platelet lysate, pooled human platelet lysate and its use for treating neurological disorders.
[5] Yan Gao, Nien-Ju Ku, Tzu-Cheng Sung, Akon Higuchi, Chi-Sheng Hung, Henry Hsin-Chung Lee, Qing-Dong Ling, Nai-Chen Cheng, Akihiro Umezawa, Lassina Barro, Thierry Burnouf, Qingsong Ye, Hao Chen. The effect of human platelet lysate on the differentiation ability of human adipose-derived stem cells cultured on ECM-coated surfaces. J Mater Chem B. 2019 Dec 7;7(45):7110-7119. doi: 10.1039/c9tb01764j.
[6] Castander-Olarieta A, Pereira C, Montalbán IA, Mendes VM, Correia S, Suárez-Álvarez S, Manadas B, Canhoto J, Moncaleán P. Proteome-Wide Analysis of Heat-Stress in Pinus radiata Somatic Embryos Reveals a Combined Response of Sugar Metabolism and Translational Regulation Mechanisms. Front Plant Sci. 2021 Apr 12; 12:631239; doi: 10.3389/fpls.2021.631239.
[7] Jennewein C, Tran N, Paulus P, Ellinghaus P, Eble JA, Zacharowski K. Novel aspects of fibrin(ogen) fragments during inflammation. Mol Med. 2011 May-Jun; 17(5-6):568-73; doi: 10.2119/molmed.2010.00146.

## Claims

1. A stable human platelet lysate composition comprising less than 5 µg/ml of fibrinogen and a mass ratio of PDGF-BB growth factor/fibrinogen content not less than 6 ng_{growth factor}/µg_{fibrinogen}; preferably the composition is a heparin-free composition and/or a xeno-free composition.

2. The stable human platelet lysate composition according to any of the previous claims comprising less than 4 µg/ml of fibrinogen, preferably less than 3 µg/ml of fibrinogen.

3. The stable human platelet lysate composition according to any of the previous claims wherein the mass ratio of PDGF-BB growth factor/fibrinogen content is not less than 8 ng_{growth factor}/µg_{fibrinogen}; preferably not less than 10 ng_{growth factor}/µg_{fibrinogen}.

4. The stable human platelet lysate composition according to the previous claim wherein growth factor is selected from a list consisting of: BDNF, EGF, FGF-2, GM-CSF, HGF, IL-1β, IL-8, PDGF-AB, PDGF-BB, VEGF and combinations thereof.

5. The stable human platelet lysate composition according to any of the previous claims wherein the mass ratio of growth factor; wherein the growth factor is selected from BDNF, EGF, VEGF-A, or combinations thereof; ranges from 0.5-2.0 nggrowth _{factor}/µg_{fibrinogen}, preferably 1.0-1.5 ng_{growth factor}/µg_{fibrinogen}.

6. The stable human platelet lysate composition according to any of the previous claims wherein the mass ratio of growth factor; wherein the growth factor is selected from FGF-2, GM-CSF, HGF, IL-1β, or combinations thereof; ranges from 0.05-0.5 nggrowth _{factor}/µg_{fibrinogen}, preferably 0.1-0.4 nggrowth _{factor}/µg_{fibrinogen}.

7. The stable human platelet lysate composition according to any of the previous claims wherein the mass ratio of growth factor; wherein the growth factor is selected from PDGF-AB, PDGF-BB, or combinations thereof; ranges from 4-12 nggrowth _{factor}/µg_{fibrinogen}, preferably 6-8 ng_{growth factor}/µg_{fibrinogen}.

8. The stable human platelet lysate composition according to any of the previous claims wherein the composition has a clear yellow colour, without visible particles detected by visual observation, more preferably without insoluble visible particles.

9. The stable human platelet lysate composition according to any of the previous claims wherein the clarity and opalescence degree, measured by nephelometry, less than 400 NTU; preferably less than 350 NTU; more preferably less than 300 NTU.

10. The stable human platelet lysate composition according to any of the previous claims wherein the optical density is inferior to 1, preferably inferior to 0.9, more preferably inferior to 0.8.

11. The stable human platelet lysate composition according to any of the previous claims wherein the number of sub-visible particles up to 25 µm of size is inferior to 2.0 × 10⁷ particles/mL, preferably inferior to 1.0 × 10⁷ particles/mL, more preferably inferior to 5.0 × 10⁶ particles/mL.

12. The stable human platelet lysate composition according to any of the previous claims wherein the amount of fibrinogen beta chains is not detectable by proteomic analysis using LC-MS.

13. The stable human platelet lysate composition according to any of the previous claims wherein said composition is stable upon storage for up to 6 months in refrigerated conditions at -80°C to 5°C, preferably -20°C to 4°C.

14. The stable human platelet lysate composition according to any of the previous claims for use in medicine or veterinary and /or for use as raw material in cell culture supplementation and/or for use as raw material in the production of advanced therapy medicinal product.

15. Method for obtaining a stable human platelet lysate comprising the following steps:
obtaining frozen human platelet units;
submitting said human platelet units to thawing / freezing cycles to disrupt the platelets membrane;
adding silica and calcium chloride to the mixture obtained in the previous step to induce clot formation;
incubating the samples at a temperature ranging from 35 to 39°C, for up to 1 to 4 hours, preferentially 2 hours;
centrifugation of the incubated samples and collection of the obtained supernatant;
purification of the supernatant in order to obtain a stable human platelet lysate; preferably a stable heparin-free human platelet lysate; optionally comprising the addition of 0.005 - 0.04 % (w/v) of silica combined with 0.5-1.0 % (w/v) of calcium chloride.
